(19) Europäisches Patentamt
European Patent Office
Office européen des brevets

(11) Veröffentlichungsnummer: **0 013 959**
**B1**

(12) # EUROPÄISCHE PATENTSCHRIFT

(45) Veröffentlichungstag der Patentschrift: **07.07.82**

(21) Anmeldenummer: **80100272.6**

(22) Anmeldetag: **21.01.80**

(51) Int. Cl.³: **C 07 J 63/00,** A 61 K 31/19, A 61 K 31/215 // C12P33/02, C12P33/08

(54) **Neue D-Homosteroide, Verfahren zu deren Herstellung und diese D-Homosteroide enthaltende Arzneimittel.**

(30) Priorität: **24.01.79 CH 707/79**

(43) Veröffentlichungstag der Anmeldung: **06.08.80 Patentblatt 80/16**

(45) Bekanntmachung des Hinweises auf die Patenterteilung: **07.07.82 Patentblatt 82/27**

(84) Benannte Vertragsstaaten: **AT BE CH DE FR GB IT LU NL SE**

(56) Entgegenhaltungen:
**EP-A-0 005 523**
**FR-A-2 122 539**
**FR-A-2 305 976**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Alig, Leo, Dr., Liebrütistrasse 32, CH-4303 Kaiseraugst (CH)**
Erfinder: **Fürst, Andor, Dr., Magnolienpark 14, CH-4052 Basel (CH)**
Erfinder: **Müller, Marcel, Dr., Quellenweg 10, CH-4402 Frenkendorf (CH)**
Erfinder: **Kerb, Ulrich, Dr., Prinzregentenstrasse 7, D-1000 Berlin 31 (DE)**
Erfinder: **Kieslich, Klaus, Dr., Strasse zum Löwen 4, D-1000 Berlin 39 (DE)**
Erfinder: **Wiechert, Rudolf, Prof. Dr., Petzowerstrasse 8a, D-1000 Berlin 39 (DE)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 013 959 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue D-Homosteroide, Verfahren zu deren Herstellung und diese D-Homosteroide enthaltende Arzneimittel

Die Erfindung betrifft neue D-Homosteroide der Formel

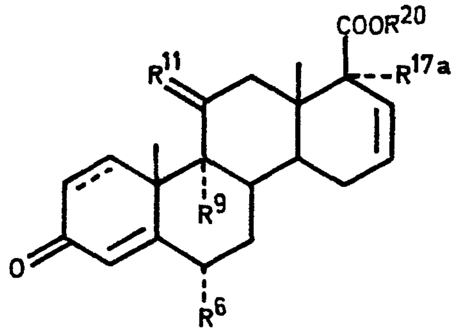

I

in der $R^6$ Wasserstoff, Fluor, Chlor oder Methyl; $R^9$ Waserstoff, Fluor, Chlor oder Brom; $R^{11}$ Oxo oder (α-H, β-OH) bedeutet, wenn $R^9$ Waserstoff ist, oder Oxo, (α-H, β-OH), (α-H, β-Fluor) oder (α-H, β-Chlor) bedeutet, wenn $R^9$ = F, Cl oder Br ist, wobei für die Ordnungszahlen der Substituenten $R^9$ und $R^{11}$ von 9,11-Dihalogenverbindungen $R^{11} \leqslant R^9$ gilt; $R^{17a}$ Acyloxy und $R^{20}$ Niederalkyl oder Halo-niederalkyl und die punktierte 1,2-Bindung eine fakultative C-C-Bindung bedeuten.

Eine Acyloxygruppe kann sich von einer gesättigten oder ungesättigten aliphatischen Carbonsäure, einer cycloaliphatischen, araliphatischen oder einer aromatischen Carbonsäure mit vorzugsweise bis zu 15 C-Atomen ableiten. Beispiele solcher Säuren sind: Ameisensäure, Essigsäure, Pivalinsäure, Propionsäure, Buttersäure, Capronsäure, Oenanthsäure, Undecylensäure, Ölsäure, Cyclopentylpropionsäure, Cyclohexylpropionsäure, Phenylessigsäure, Salicylsäure, Acetylsalicylsäure und Benzoesäure. Besonders bevorzugt sind $C_{1-7}$-Alkanoyloxygruppen. Nieder-alkylgruppen können gerad- oder verzweigtkettig sein und 1-16 C-Atome enthalten. Besonders bevorzugt sind Nieder-alkylgruppen mit 1-4 Kohlenstoffatomen, insbesondere Methyl und Äthyl. In gleicher Weise ist die Bezeichnung Halo-niederalkyl zu verstehen. Der Ausdruck «Halo» umfasst, sofern er nicht ausdrücklich enger definiert ist, Fluor, Chlor, Brom und Jod. Beispiele von Halo-nieder-alkylresten sind Fluormethyl, Chlormethyl, Brommethyl, β-Fluoräthyl, β-Chloräthyl und β-Bromäthyl.

Bei 9,11-Dihalogenverbindungen der Formel I soll, wie erwähnt, das Halogenatom in 11-Stellung eine kleinere oder höchstens gleich grosse Ordnungszahl wie das 9-ständige Halogenatom besitzen. Bei Verbindungen der Formel I mit $R^9$ = H soll dagegen $R^{11}$ nur Oxo oder (α-H, β-OH) sein.

Eine bevorzugte Gruppe von Verbindungen der Formel I sind die, in denen $R^{11}$ (α-H, β-OH) darstellt. Weiterhin sind solche Verbindungen bevorzugt, in denen $R^9$ Wasserstoff oder Fluor darstellt. Eine andere, bevorzugte Untergruppe stellen die Verbindungen der Formel I, worin $R^{17a}$ $C_{1-7}$-Alkanoyloxy und $R^{20}$ Methyl, Chlormethyl oder Fluormethyl ist, dar.

Beispiele von erfindungsgemässen Verbindungen sind:

17α-Acetoxy-11β-hydroxy-3-oxo-D-homoandrosta-1,4,16-trien-17aβ-carbonsäuremethylester,

11β-Hydroxy-3-oxo-17a-propionyloxy-D-homoandrosta-1,4,16-trien-17aβ-carbonsäurechlormethylester,

11β-Hydroxy-3-oxo-17a-propionyloxy-D-homoandrosta-1,4,16-trien-17aβ-carbonsäurefluormethylester,

17a-Acetoxy-3,11-dioxo-D-homoandrosta-1,4,-16-trien-17aβ-carbonsäuremethylester,

9-Chlor-11β-fluor-3-oxo-17a-propionyloxy-D-homoandrosta-1,4,16-trien-17aβ-carbonsäurechlormethylester,

9-Brom-11β-hydroxy-3-oxo-17a-propionyloxy-D-homoandrosta-1,4,16-trien-17aβ-carbonsäurechlormethylester,

11β-Hydroxy-6α-methyl-3-oxo-17a-propionyloxy-D-homoandrosta-1,4,16-trien-17aβ-carbonsäuremethylester,

6α-Fluor-11β-hydroxy-3-oxo-17a-propionyloxy-D-homoandrosta-1,4,16-trien-17aβ-carbonsäurefluormethylester,

9-Fluor-11β-hydroxy-3-oxo-17a-propionyloxy-D-homoandrosta-1,4,16-trien-17aβ-carbonsäurechlormethylester,

17a-(o-Acetoxy-benzoyloxy)-11β-hydroxy-3-oxo-D-homoandrosta-1,4,16-trien-17aβ-carbonsäurefluormethylester,

17a-Butyryloxy-11β-hydroxy-3-oxo-D-homoandrosta-1,4,16-trien-17aβ-carbonsäureäthylester,

9,11β-Dichlor-3-oxo-17a-propionyloxy-D-homoandrosta-1,4,16-trien-17aβ-carbonsäurechlormethylester,

9-Fluor-11β-hydroxy-3-oxo-17a-propionyloxy-D-homoandrosta-1,4,16-trien-17aβ-carbonsäuremethylester,

9-Fluor-11β-hydroxy-3-oxo-17a-propionyloxy-D-homoandrosta-1,4,16-trien-17aβ-carbonsäurefluormethylester,

17a-Acetoxy-6α,9-difluor-11β-hydroxy-3-oxo-D-homoandrosta-1,4,16-trien-17aβ-carbonsäuremethylester,

17a-Acetoxy-11β-hydroxy-3-oxo-D-homoandrosta-4,16-dien-17aβ-carbonsäuremethylester,

11β-Hydroxy-3-oxo-17a-propionyloxy-D-homoandrosta-4,16-dien-17aβ-carbonsäurechlormethylester,

11β-Hydroxy-3-oxo-17a-propionyloxy-D-homoandrosta-4,16-dien-17aβ-carbonsäurefluormethylester,

17a-Acetoxy-3,11-dioxo-D-homoandrosta-4,16-dien-17aβ-carbonsäuremethylester,

9-Chlor-11β-fluor-3-oxo-17a-propionyloxy-D-homoandrosta-4,16-dien-17aβ-carbonsäurechlormethylester,

9-Brom-11β-hydroxy-3-oxo-17a-propionyloxy-D-homoandrosta-4,16-dien-17aβ-carbonsäurechlormethylester,

11β-Hydroxy-6α-methyl-3-oxo-17a-propionyloxy-D-homoandrosta-4,16-dien-17aβ-carbonsäuremethylester,

6α-Fluor-11β-hydroxy-3-oxo-17a-propionyloxy-D-homoandrosta-4,16-dien-17aβ-carbonsäurefluormethylester,

9-Fluor-11β-hydroxy-3-oxo-17a-propionyloxy-D-homoandrosta-4,16-dien-17aβ-carbonsäurechlormethylester,

17a-(o-Acetoxybenzoyloxy)-11β-hydroxy-3-oxo-D-homoandrosta-4,16-dien-17aβ-carbonsäurefluormethylester,

17a-Butyryloxy-11β-hydroxy-3-oxo-D-homoandrosta-4,16-dien-17aβ-carbonsäureäthylester.

Die D-Homosteroide der Formel I können erfindungsgemäss dadurch hergestellt werden, dass man

a) ein 1,2-gesättigtes D-Homosteroid der Formel I in 1,2-Stellung dehydriert, oder

b) in einem D-Homosteroid der Formel

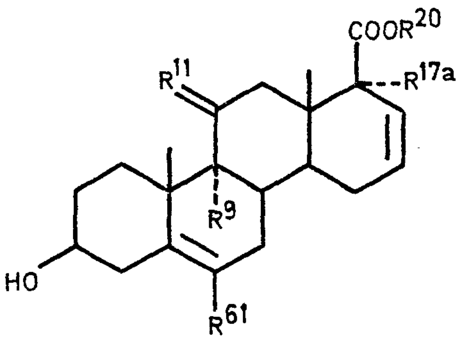

II

die 3-Hydroxy-$\Delta^5$-Gruppierung zur 3-Keto-$\Delta^4$-Gruppierung oxydiert, oder

c) ein D-Homosteroid der Formel

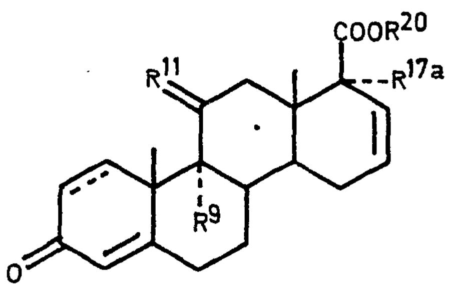

III

in 6-Stellung fluoriert oder chloriert und ein erhaltenes 6β-Isomer gewünschtenfalls zum 6α-Isomer isomerisiert, oder

d) ein D-Homosteroid der Formel

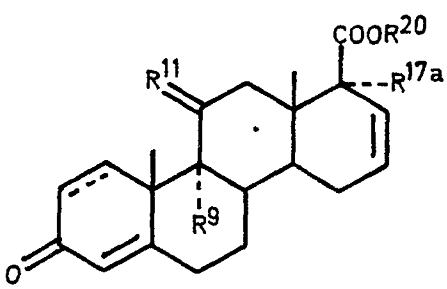

III

in 6-Stellung methyliert, oder

e) ein D-Homosteroid der Formel

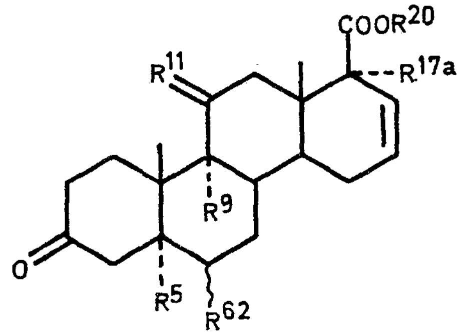

IV

einer $HR^5$-Abspaltung unterwirft, oder

f) an die 9,11-Doppelbindung eines D-Homosteroids der Formel

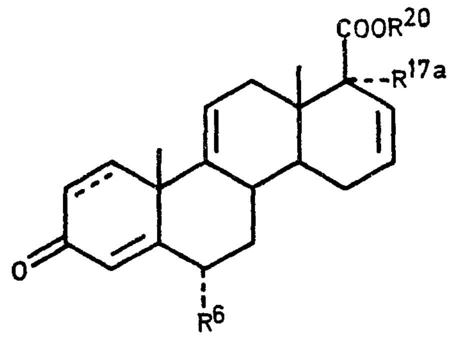

V

Chlor, ClF, BrF, BrCl, unterchlorige oder unterbromige Säure anlagert, oder

g) ein D-Homosteroid der Formel

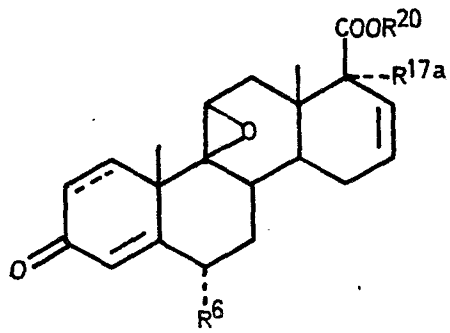

VI

mit Fluor-, Chlor- oder Bromwasserstoff behandelt, oder

h) ein D-Homosteroid der Formel

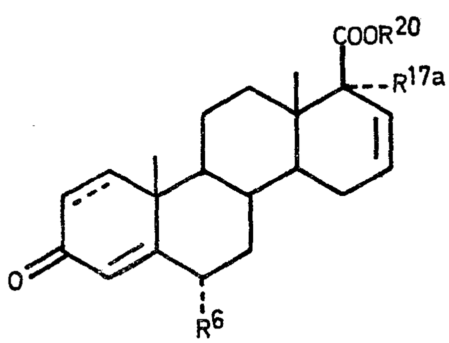

VII

mittels Mikroorganismen oder daraus gewonnenen Enzymen in 11-Stellung hydroxyliert, oder

i) die 11-Ketogruppe eines D-Homosteroids der Formel

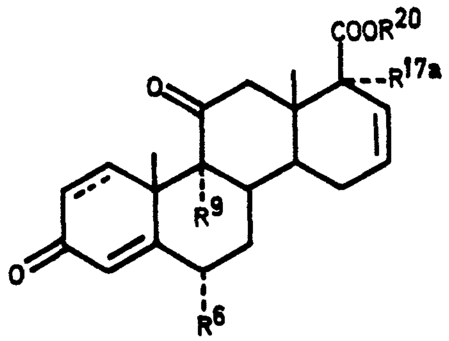

VIII

zur 11β-Hydroxygruppe reduziert, oder

j) die 11-Hydroxygruppe eines D-Homosteroids der Formel

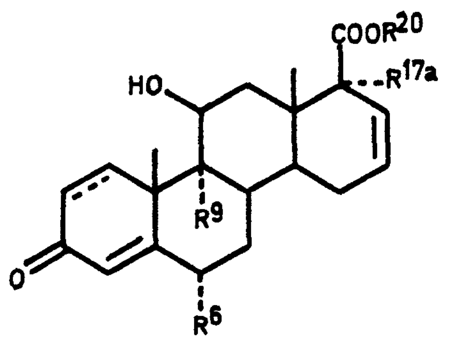

IX

zur Ketogruppe oxydiert, oder

k) die 17aα-Hydroxygruppe in einem D-Homosteroid der Formel

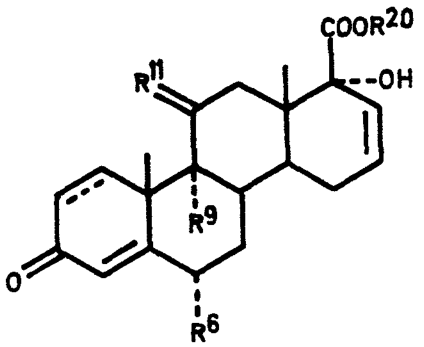

X

acyliert, oder

l) die Gruppe $COOR^{201}$ in einem D-Homosteroid der Formel

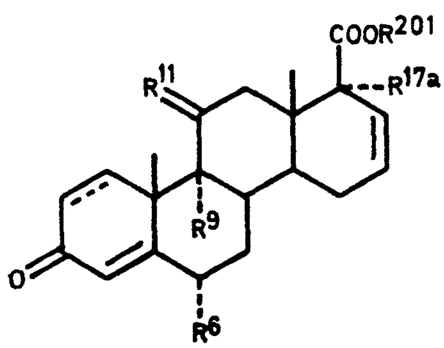

XI

funktionell abwandelt, oder

m) die 1,2-Doppelbindung in einem D-Homosteroid der Formel

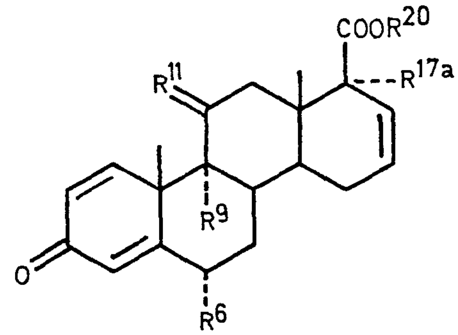

XII

hydriert,

wobei in den vorstehenden Formeln $R^6$, $R^9$, $R^{11}$, $R^{17a}$ und $R^{20}$ und die punktierte 1,2-Bindung die bereits angegebene Bedeutung haben; $R^5$ Hydroxy, Fluor, Chlor oder Brom, $R^{61}$ Wasserstoff oder Methyl; $R^{62}$ Fluor, Chlor oder Methyl und $R^{201}$ Wasserstoff oder eine Gruppe $R^{20}$ darstellen.

Die 1,2-Dehydrierung eines D-Homosteroids der Formel I (Verfahrensvariante a) kann in an sich bekannter Weise, z.B. auf mikrobiologischem Wege oder mittels Dehydrierungsmitteln, wie Jodpentoxyd, Perjodsäure oder Selendioxyd, 2,3-Dichlor-5,6-dicyanobenzochinon, Chloranil oder Bleitetraacetat vorgenommen werden. Geeignete Mikroorganismen für die 1,2-Dehydrierung sind beispielsweise Schizomyceten, insbesondere solche der Genera Arthrobacter, z.B. A. simplex ATCC 6946; Bacillus, z.B. B. lentus ATCC 13805 und B. sphaericus ATCC 7055; Pseudomonas, z.B. P. aeruginosa IFO 3505; Flavobacterium, z.B. F. flavenscens IFO 3058; Lactobacillus, z.B. L. brevis IFO 3345 und Nocardia, z.B. N. opaca ATCC 4276.

Die Oxydation einer Verbindung der Formel II (Verfahrensvariante b) kann nach an sich bekannten Methoden, wie z.B. nach Oppenauer, mittels Aluminiumisopropylat, oder mit Oxydationsmitteln wie Chromtrioxyd (z.B. Jones'Reagens), oder nach Pfitzner-Moffatt mittels Dimethylsulfoxyd/Dicyclohexylcarbodiimid (wobei das primär erhaltene $\Delta^5$-3-Keton anschliessend zum $\Delta^4$-3-Keton isomerisiert werden muss); oder mittels Pyridin/$SO_3$ vorgenommen werden.

Die Halogenierung eines Steroids der Formel III in 6-Stellung (Verfahrensvariante c) kann in an sich bekannter Weise durchgeführt werden. Ein 6,7-gesättigtes D-Homosteroid der Formel III kann durch Umsetzung mit einem Halogenierungsmittel, wie einem N-Chloramid oder -imid (z.B. N-Chlorsuccinimid) oder mit elementarem Chlor halogeniert werden [vgl. J. Am. Chem. 72, 4534 (1950)]. Die Halogenierung in 6-Stellung wird vorzugsweise dadurch vorgenommen, dass man ein 6,7-gesättigtes D-Homosteroid der Formel III in einen 3-Enolester oder 3-Enoläther, z.B. das 3-Enolacetat, überführt und danach mit Chlor [vgl. J. Am. Chem. Soc. 82, 1230 (1960)]; mit einem N-Chlorimid [vgl. J. Am. Chem. Soc. 82, 1230 (1960); 77, 3827 (1955)] oder Perchlorylfluorid [vgl. J. Am. Chem. Soc. 81, 5259 (1959); Chem. and Ind. 1959, 1317] umsetzt. Als Fluorierungsmittel kommt weiterhin Trifluormethylhypofluorit in Betracht.

Sofern bei den vorstehend beschriebenen Halogenierungen Isomerengemische, d.h. Gemische von 6α- und 6β-Halogensteroiden gebildet werden, können diese nach bekannten Methoden, wie Chromatographie, in die reinen Isomeren getrennt werden.

Die Isomerisierung einer 6β-Fluor- oder Chlorverbindung kann durch Behandlung mit einer Säure, insbesondere einer Mineralsäure, wie Salzsäure oder Bromwasserstoffsäure, in einem Lösungsmittel, z.B. Dioxan oder Eisessig, vorgenommen werden.

Die Methylierung gemäss Verfahrensvariante d) kann z.B. dadurch bewerkstelligt werden, dass man die Ausgangsverbindung der Formel III in einen 3-Enoläther überführt (z.B. durch Behandlung mit einem Orthoameisensäureester, wie Äthyl-orthoformiat, in Gegenwart einer Säure, wie p-Toluolsulfonsäure, gegebenenfalls unter Zusatz des entsprechenden Alkohols; oder durch Behandlung der Ausgangsverbindung III mit einem Dialkoxypropan, z.B. 2,2-Dimethoxypropan in Methanol-Dimethylformamid in Gegenwart von p-Toloulsulfonsäure) und den Enoläther mit einem Tetrahalomethan, z.B. $CBr_4$, $CCl_2Br_2$ oder $CCl_3Br$ zum Trihalomethyl-$\Delta^4$-3-keton umsetzt. Das Trihalomethyl-$\Delta^4$-3-keton kann mit Basen, wie Collidin, zum Dihalomethylen-$\Delta^4$-3-keton dehydrohalogeniert werden, das wiederum durch katalytische Hydrierung unter milden Bedingungen, z.B. mit einem $Pd/SrCO_3$-Katalysator, in das 6α-Methyl-$\Delta^4$-3-keton übergeführt werden kann.

Eine andere Methylierungsmethode besteht darin, dass man ein 1,2-gesättigtes D-Homosteroid der Formel III wie oben beschrieben, in einen 3-Enoläther überführt und diesen in an sich bekannter Weise zu einem entsprechenden 6-Formylderivat umsetzt, die Formylgruppe mit Natriumborhydrid zur Hydroxymethylgruppe reduziert und das Reaktionsprodukt schliesslich unter Spaltung des Enoläthers dehydratisiert, wobei ein D-Homosteroid der Formel

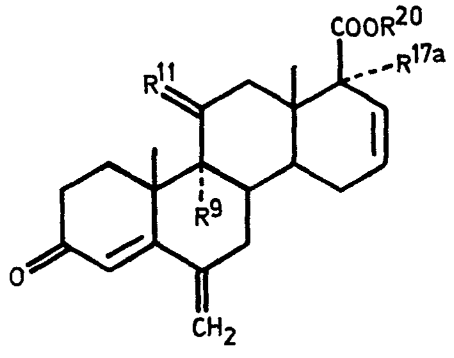

XIII

in der $R^9$, $R^{11}$, $R^{17a}$ und $R^{20}$ die oben genannten Bedeutungen haben, erhalten wird.

Solche 6-Methylen-Zwischenprodukte können auch durch Überführung von Verbindungen III in ein 3-Enamin, z.B. das 3-Pyrrolidinium-enamin, Hydroxymethylierung mit Formaldehyd und Wasserabspaltung mittels Säuren, wie p-Toluolsulfonsäure, hergestellt werden. Die so erhaltenen 6-Methylen-D-homosteroide der Formel XIII können in an sich bekannter Weise katalytisch, d.h. mittels der bekannten Hydrierungskatalysatoren, zu den entsprechenden 6-Methylverbindungen hydriert werden.

Eine $HR^5$-Abspaltung aus einer Verbindung der Formel IV (Verfahrensvariante e), d.h. eine Dehydratisierung oder eine Dehydrohalogenierung, kann in an sich bekannter Weise erfolgen. Die Dehydratisierung kann durch Behandlung mit einer Säure, z.B. einer Mineralsäure, wie Salzsäure, oder mit einer Base durchgeführt werden. Die Dehydrohalogenierung kann mittels Basen, z.B. organischen Basen, wie Pyridin, bewerkstelligt werden.

Zwecks Durchführung der Verfahrensvariante f) und g) löst man das Ausgangsmaterial V bzw. VI zweckmässigerweise in einem geeigneten Lösungsmittel, z.B. einem Äther, wie Tetrahydrofuran oder Dioxan, einem chlorierten Kohlenwasserstoff, wie Methylenchlorid oder Chloroform, oder einem Keton, wie Aceton, und lässt das anzulagernde Reagens einwirken. Unterchlorige oder unterbromige Säure wird zweckmässigerweise im Reaktionsgemisch selbst erzeugt; z.B. aus N-Brom- oder N-Chloramiden oder -imiden, wie N-Chlorsuccinimid oder N-Bromacetamid, und einer starken Säure, vorzugsweise Perchlorsäure. Die Variante g) wird vorzugsweise zur Herstellung von 9-Fluor-11-hydroxy-D-homosteroiden der Formel I benutzt.

Die Hydroxylierung einer Verbindung der Formel VII gemäss Verfahrensvariante h) kann mittels für die mikrobielle 11-Hydroxylierung von Steroiden an sich bekannter Methoden bewerkstelligt werden. Hierfür kommen Mikroorganismen der taxonomischen Einheiten Fungi und Schizomycetes, insbesondere der Untereinheiten Ascomycetes, Phycomycetes, Basidiomycetes und Actinomycetales, in Betracht. Es können auch auf chemischem Wege, z.B. durch Behandlung mit Nitrit, oder auf physikalischem Wege, z.B. durch Bestrahlung, erzeugte Mutanten sowie aus den Mikroorganismen erhaltene zellfreie Enzympräparate verwendet werden. Geeignete Mikroorganismen für die 11β-Hydroxylierung sind insbesondere solche der Genera Curvalaria, z.B. C. lunata NRRL 2380 und NRRL 2178; ATCC 13633, 13432, 14678, IMJ 77007, IFO 2811; Absidia, z.B. A. coerula IFO 4435; Colletotrichum, z.B. C. pisi ATCC 12520; Pellicularia, z.B. P. filamentosa IFO 6675; Streptomyces, z.B. S. fradiae ATCC 10745; Cunninghamella, z.B. C. bainieri ATCC 9244, C. verticillata ATCC 8983, C. elegans NRRL 1392 und ATCC 9245, C. blakesleeana ATCC 8688, 8688a, 8688b, 8983, und C. echinulata ATCC 8984; Pycnosporium, z.B. sp. ATCC 12231; Verticillium, z.B. V. theobromae CBS 39858; Aspergillus, z.B. A. quadrilineatus JAM 2763; Trichothecium, z.B. T. roseum ATCC 12519; und Phoma, z.B. sp. ATCC 13145.

Zur Durchführung der Verfahrensvariante i) wird zunächst die Ketogruppe der Verbindung VIII in 3-Stellung geschützt. Die 3-Ketogruppe

kann durch Ketalisierung oder, falls eine 1,2-Doppelbindung anwesend ist, auch durch Bildung eines Enamins geschützt werden. Diese Schutzgruppen können durch saure Hydrolyse wieder entfernt werden. Ein $\Delta^{1,4}$-3-Keton kann mit einem sek. Amin in Gegenwart von $TiCl_4$ in ein $\Delta^{1,3,5}$-3-Enamin übergeführt werden. Die Reduktion der 11-Ketogruppe der so geschützten Verbindung kann mit komplexen Metallhydriden, wie Lithiumaluminiumhydrid, Natriumborhydrid oder Diisobutylaluminiumhydrid erfolgen. Bei Verwendung von $NaBH_4$ in THF (Chem. and Ind. 1977, 982) kann die 11-Ketogruppe ohne intermediären Schutz der $\Delta^{1,4}$-3-Ketogruppe selektiv reduziert werden.

Für die Oxydation gemäss Verfahrensvariante j) kommen Oxydationsmittel wie Chromsäure, z.B. $CrO_3$/Schwefelsäure in Aceton oder $CrO_3$/Pyridin in Betracht.

Die Acylierung einer 17a-Hydroxygruppe (Verfahrensvariante k) kann in an sich bekannter Weise, z.B. durch Behandlung mit einem Acylierungsmittel, wie einem Acylchlorid oder -anhydrid, in Gegenwart eines säurebindenden Mittels, z.B. Pyridin oder Triäthylamin, oder in Gegenwart eines starken Säurekatalysators, z.B. p-Toluolsulfonsäure, durchgeführt werden. Als Lösungsmittel für die Acylierung kommen nicht-hydroxylgruppenhaltige organische Lösungsmittel, z.B. chlorierte Kohlenwasserstoffe, wie Methylenchlorid, oder Kohlenwasserstoffe, wie Benzol, in Betracht. Es ist auch möglich, ein 17aα-Hydroxy-17aβ-carbonsäure-D-homosteroid der Formel X mit einem entsprechenden Carbonsäureanhydrid zunächst in ein gemischtes Anhydrid der Steroidcarbonsäure zu überführen und dieses gemischte Anhydrid sauer oder basisch (z.B. mit wässriger Essigsäure bzw. wässigem Pyridin) zu behandeln, wodurch man das gewünschte 17aα-Acyloxyderivat der Ausgangsverbindung X erhält.

Die funktionelle Abwandlung der Gruppe $-COOR^{201}$ (Verfahrensvariante 1) kann z.B. in einer Veresterung einer 20-Carboxylgruppe, einer Umesterung einer veresterten 20-Carboxylgruppe oder durch Halogenaustausch bestehen. Alle diese Reaktionen können nach an sich bekannten Methoden durchgeführt werden. Die Veresterung kann z.B. durch Behandlung der freien Säure mit einem Diazoalkan, z.B. Diazomethan in Äther; oder mit einem O-Alkyl-N,N'-dicyclohexylisoharnstoff in einem aprotischen Lösungsmittel; oder durch Umsetzung eines Salzes der Säure, z.B. eines Alkalisalzes, mit einem Alkylhalogenid oder -sulfat, z.B. Methyl- oder Äthyljodid bzw. Dimethyl- oder Diäthylsulfat, erreicht werden.

Die Umesterung einer veresterten Carboxylgruppe, z.B. der Austausch einer durch $R^{201}$ dargestellten Alkylgruppe gegen eine andere Alkylgruppe kann durch Umsetzung mit dem entsprechenden Alkohol in Gegenwart eines sauren Katalysators, wie Perchlorsäure, durchgeführt werden. Verbindungen, in denen $R^{201}$ eine Halogen-substituierte Alkylgruppe darstellt, können z.B. dadurch hergestellt werden, dass man ein Salz einer D-Homosteroidcarbonsäure der Formel XI mit einem Dihaloalkan, wie z.B. Chlorjodmethan oder Methylenchlorid, oder einem Sulfonyloxyalkylhalogenid zu einem Sulfonyloxyalkylester umsetzt und letzteren mit einem Alkali- oder Erdalkalihalogenid, z.B. Lithiumchlorid in Dimethylformamid, behandelt, um einen Halogenalkylsubstituenten $R^{201}$ zu erhalten. Halogenalkylester können auch dadurch erhalten werden, dass man die D-Homosteroid-carbonsäure mit einem entsprechenden Aldehyd in Gegenwart von Halogenwasserstoff, zweckmässig in Gegenwart eines Katalysators, wie $ZnCl_2$, umsetzt.

Die Hydrierung einer 1,2-Doppelbindung in einem D-Homosteroid der Formel XII (Verfahrensvariante m) kann katalytisch, z.B. mit Pd oder Tris-(triphenylphosphin)-rhodiumchlorid vorgenommen werden.

Die Ausgangsstoffe können, soweit sie nicht bekannt oder nachstehend beschrieben sind, in Analogie zu bekannten oder in den Beispielen beschriebenen Methoden dargestellt werden.

Die D-Homosteroide der Formel I sind endokrin, insbesondere antiinflammatorisch wirksam. Dabei weisen sie ein gutes Verhältnis der antiinflammatorischen Wirkung zu Effekten mineralo- oder glucocorticoider Natur auf. In der folgenden Tabelle sind für zwei Verbindungen die Ergebnisse von zwei Standard-Tests zusammengefasst, die die Wirkungen der Verbindungsklasse belegen. Die durchgeführten Tests lassen sich folgendermassen beschreiben:

1. Mäuseohr-Test

Männlichen Mäusen (25-30 g) wurden die Prüfsubstanzen, gelöst in Crotonöl, mit einem Druck von 600 g 15 Sekunden auf das rechte Ohr aufgetragen. Das linke Ohr diente als Kontrolle. 4 Stunden später wurden die Tiere getötet, und vom behandelten und unbehandelten Ohr an gleicher Stelle mit einer Stanze Gewebe entnommen und gewogen. Ermittelt wurde die $EC_{50}$, d.h. diejenige Konzentration, bei der eine, verglichen mit einer Kontrollgruppe, 50%ige Oedemhemmung auftrat.

2. Filzpellet-Test

Weiblichen Ratten (90-110 g) wurden in Äthernarkose 2 Filzpellets unter die Haut (Scapularegion) implantiert. Die Prüfsubstanzen wurden an 4 aufeinanderfolgenden Tagen, beginnend am Tage der Implantation, oral verabreicht. Am 5. Tage wurden die Tiere getötet, das gebildete Granulom abgetragen, getrocknet und gewogen. Ermittelt wurde die $ED_{40}$, d.h. diejenige Dosis, bei der eine 40%ige Hemmung des Granulationsgewichtes auftrat.

| Verbindung \ Test | Mäuseohr $EC_{50}$ (mg/ml) | Filzpellet $ED_{40}$ (mg/kg) |
|---|---|---|
| 17aα-Acetoxy-11β-hydroxy-3-oxo-D-homoandrosta-1,4,16-trien-17β-carbonsäuremethylester | 0,03 | 6,4 |
| 17aα-Acetoxy-9-fluor-11β-hydroxy-3-oxo-D-homoandrosta-1,4,16-trien-17β-carbonsäuremethylester | 0,01 | 6,0 |

Die Verfahrensprodukte können als Heilmittel in Form pharmazeutischer Präparate mit direkter oder verzögerter Freigabe des Wirkstoffs in Mischung mit einem für die enterale, perkutane oder parenterale Applikation geeigneten organischen oder anorganischen inerten Trägermaterial, wie z.B. Wasser, Gelatine, Gummi arabicum, Milchzucker, Stärke, Magnesiumstearat, Talk, pflanzliche Öle, Polyalkylenglykole, Vaseline, usw. verwendet werden. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffersubstanzen.

Als Richtlinie für die Zusammensetzung topischer Präparate kann etwa 0,01-1% Gehalt an einer Verbindung der Formel I gelten. Bei systemischen Präparaten können etwa 0,1-10 mg Wirkstoff pro Applikation vorgesehen werden.

Die Herstellung der Arzneimittel kann in an sich bekannter Weise erfolgen, indem man die Verbindungen der Formel I mit zur therapeutischen Verabreichung geeigneten, nicht-toxischen, an sich in solchen Präparaten üblichen festen und/oder flüssigen Trägermaterialien, wie z.B. den vorstehend genannten, vermischt und gegebenenfalls in die gewünschte Form bringt.

Im Gegensatz zu den aus der FR-A-2 305 976 bekannten Verbindungen enthalten die Verbindungen der Formel I eine Doppelbindung in 16,17-Stellung. Ferner haben letztere Verbindungen eine höhere antiinflammatorische Wirksamkeit als die entsprechenden in der FR-A-2 305 976.

In den folgenden Beispielen sind die Temperaturen in Celsiusgraden angegeben.

Beispiel 1

Ein 2-l-Erlenmeyerkolben, der 500 ml einer 30 Min. bei 120° im Autoklaven sterilisierten Nährlösung aus 6% flüssigem Stärkezucker (3% Glucose), 1% Cornsteep, 0,2% $NaNO_3$, 0,1% $KH_2PO_4$, 0,2% $K_2HPO_4$, 0,05% $MgSO_4$, 0,002% $FeSO_4$, 0,05% KCl, eingestellt auf pH 6,0, enthält, wird mit einer Lyophilkultur von Phoma Spec. ATCC 13145 beimpft und 72 Stunden bei 30° geschüttelt. Mit dieser Vorkultur wird ein 20-l-Fermenter aus rostfreiem Stahl, der mit 15 l eines bei 121° und 1,1 atü sterilisierten Mediums gleicher Zusammensetzung beimpft. Unter Zugabe eines Antischaummittels wird bei 29° unter Belüftung (15 l/Min), 0,7 atü Druck und Rühren (220 U/min) 24 Stunden germiniert. 1 Liter der Kulturbrühe wird unter sterilen Bedingungen in 14 l eines wie oben sterilisierten Mediums der gleichen Zusammensetzung übergeführt und unter gleichen Bedingungen angezüchtet. Nach 12 Stunden wird eine sterilfiltrierte Lösung von 3 g 17aα-Acetoxy-3-oxo-D-homo-4,16-androstadien-17aβ-carbonsäuremethylester in 100 ml DMF zugegeben, nach 53 Stunden Kontaktzeit wird der Fermenterinhalt über Gaze filtriert und das Filtrat zweimal mit je 10 l Methylisobutylketon ausgerührt. Das abfiltrierte Mycel wird ebenfalls mit Methylisobutylketon mehrmals gewaschen. Waschlösungen und Extrakte werden vereinigt und im Vakuum bei 50° Badtemperatur eingedampft. Der Rückstand wird zur Entfernung des Antischaummittels mehrmals mit Hexan gewaschen und aus Essigester unter Zusatz von Kohle umkristallisiert. Aus drei vereinigten Fermenteransätzen mit einem Gesamtsubstrateinsatz von 8,8 g werden 2,79 g 17aα-Acetoxy-11β-hydroxy-3-oxo-D-homoandrosta-4,16-dien-17aβ-carbonsäuremethylester, Smp. 228/229-231° erhalten. Durch Chromatographie der Mutterlaugen werden weitere 1,32 g der Verbindung erhalten (Gesamtausbeute 44,9% d. Th.).

Beispiel 2

Ein 2-l-Erlenmeyerkolben, der 500 ml einer 30 Min. bei 120° im Aoutoklaven sterilisierten Nährlösung aus 1,5% Pepton, 1,2% Cornsteep und 0,2% $MgSO_4$, eingestellt auf pH 6,5 enthält, wird mit einer Lyophilkultur von Bacillus lentus (ATCC 13805) beimpft und 24 Stunden bei 30° geschüttelt. Mit dieser Vorkultur wird dann ein 20-l-Fermenter aus rostfreiem Stahl, der 15 l eines bei 121° und 1,1 atü sterilisierten flüssigen Nährmediums aus 0,1% Pepton, 0,2% Cornsteep, 0,5% Glucose und 0,2% Hefeextrakt, eingestellt auf pH 7,0 enthält, beimpft. Nach 24 Stunden werden 0,9 l der Kultur unter sterilen Bedingungen in einen gleich grossen Fermenter übergeführt, der mit 14 l der gleichen Nährlösung beschickt und sterilisiert war. Unter Zugabe eines Antischaummittels wird bei 29° unter Belüftung und Rühren germiniert. Nach einer Anwachsphase von 6 Stunden setzt man eine sterilfiltrierte Lösung von 1,5 g 17aα-Acetoxy-11β-hydroxy-3-oxo-D-homoandrosta-4,16-dien-17aβ-carbonsäuremethylester in 50 ml DMF zu. Nach 32 Stunden Kontaktzeit wird der Fermenterinhalt zweimal mit je 10 l Methylisobutylketon extrahiert und der Extrakt im Vakuum eingedampft. Der Rückstand wird zur Entfernung des

Antischaummittels mit Hexan gewaschen und aus Essigester umkristallisiert. Aus drei Fermenteransätzen mit einem Gesamtsubstrateinsatz von 4,2 g wurden einschliesslich der durch Mutterlaugenchromatographie erhaltenen Anteile 2,4 g 17aα-Acetoxy-11β-hydroxy-3-oxo-D-homoandrosta-1,4,16-trien-17aβ-carbonsäuremethylester, Smp. 236/237-239° erhalten (57,5% d.Th.).

Beispiel 3

1,12 g 17a-Acetoxy-3-oxo-D-homoandrosta-1,-4,9(11),16-tetraen-17aβ-carbonsäuremethylester wurden in 38 ml Dioxan mit 7,7 ml Wasser, 0,58 g N-Bromacetamid und 3,85 ml 10%iger Perchlorsäure versetzt und 30 Minuten bei 25° gerührt. Nach Zugabe von 2 g Natriumsulfit wurde das Lösungsmittel im Vakuum abgedampft. Der Rückstand wurde in Methylenchlorid mit Wasser und verdünnter Kochsalzlösung gewaschen. Die Methylenchloridlösung wurde getrocknet und im Vakuum eingedampft. Aus Aceton-Hexan erhielt man 17a-Acetoxy-9-brom-11β-hydroxy-3-oxo-D-homoandrosta-1,4,16-trien-17aβ-carbonsäuremethylester, Smp. 213°, $[\alpha]_D$ −50° (c = 0,1% in Dioxan); $\varepsilon_{242}$ = 14540. Das Ausgangsmaterial erhält man aus 17a-Acetoxy-11β-hydroxy-3-oxo-D-homoandrosta-1,4,16-trien-17aβ-carbonsäuremethylester durch Behandlung mit Methansulfochlorid und Schwefeldioxid in Pyridin und Dimethylformamid.

Beispiel 4

953 mg 17a-Acetoxy-9,11β-epoxy-3-oxo-D-homo-9β-androsta-1,4,16-trien-17aβ-carbonsäuremethylester wurden in 4,3 ml Toluol und 2,9 ml Pyridin. 5HF 5½ Stunden bei 30° gerührt. Das Reaktionsgemisch wurde mit Methylenchlorid verdünnt, mit Wasser, verdünnter Natriumbicarbonatlösung und verdünnter Kochsalzlösung neutralgewaschen, getrocknet und im Vakuum eingedampft. Nach Chromatographie an Kieselgel erhielt man aus Aceton-Hexan 17a-Acetoxy-9-fluor-11β-hydroxy-3-oxo-D-homoandrosta-1,4,-16-trien-17aβ-carbonsäuremethylester, Smp. 257°, $[\alpha]_D$ −114° (c = 0,1% in Dioxan), $\varepsilon_{239}$ = 15400. Das Ausgangsmaterial erhält man aus 17a-Acetoxy-9-brom-11β-hydroxy-3-oxo-D-homoandrosta-1,4,16-trien-17aβ-carbonsäuremethylester und Kaliumcarbonat in Aceton am Rückfluss.

Beispiel 5

In zu Beispiel 3 analoger Weise erhält man aus dem 17a-Acetoxy-3-oxo-D-homoandrosta-4,9(11),16-trien-17aβ-carbonsäuremethylester, Smp. 201-202°C, $[\alpha]_D$ −152° (Dioxan c = 0,1%), UV: $\varepsilon_{238}$ = 17 890,

den 17a-Acetoxy-9-brom-11β-hydroxy-3-oxo-D-homoandrosta-4,16-dien-17aβ-carbonsäuremethylester, Smp. 154-155°C, $[\alpha]_D$ −39° (c = 0,1% in Dioxan), UV: $\varepsilon_{243}$ = 15 530.

Beispiel 6

In zu Beispiel 4 analoger Weise erhält man aus dem 17a-Acetoxy-9,11β-epoxy-3-oxo-D-homo-9β-androsta-4,16-dien-17aβ-carbonsäuremethylester, Smp. 213-214°, $[\alpha]_D$ −201° (c = 0,1% in Dioxan), UV: $\varepsilon_{241}$ = 15 400,

den 17a-Acetoxy-9-fluor-11β-hydroxy-3-oxo-D-homoandrosta-4,16-dien-17aβ-carbonsäuremethylester, Smp. 192-193°, $[\alpha]_D$ −100°, (c = 0,1% in Dioxan), UV: $\varepsilon_{238}$ = 17 060.

Beispiel 7

450 mg 9-Fluor-11β-hydroxy-3-oxo-17α-propionyloxy-D-homoandrosta-4,16-dien-17aβ-carbonsäure und 450 mg Natriumhydrogencarbonat wurden in 34 ml Dimethylacetamid und 11,5 ml Methylenchlorid 20 Stunden bei 60°C am Rückfluss gekocht. Das filtrierte Reaktionsgemisch wurde im Vakuum eingedampft. Der Rückstand wurde in Methylenchlorid aufgenommen und mit Wasser gewaschen. Die Methylenchloridlösung wurde getrocknet und in Vakuum eingedampft. Chromatographie des Rohprodukts an Kieselgel gab 206 mg 9-Fluor-11β-hydroxy-3-oxo-17a-propionyloxy-D-homoandrosta-4,16-dien-17aβ-carbonsäurechlormethylester, Smp. 176-177°C, $[\alpha]_D$ −56° (c = 0,1% in Dioxan), UV: $\varepsilon_{238}$ = 14 540.

Herstellung des Ausgangsmaterials:

Durch Behandlung von 17a-Acetoxy-9-fluor-11β-hydroxy-3-oxo-D-homoandrosta-4,16-dien-17aβ-carbonsäuremethylester mit Kaliumhydroxid in Methoxyäthanol erhält man 9-Fluor-11β,17a-dihydroxy-3-oxo-D-homoandrosta-4,16-dien-17aβ-carbonsäure, Smp. 222-224°C. Daraus erhält man durch Umsetzung mit Propionylchlorid in Dichlormethan in Gegenwart von Triäthylamin die 9-Fluor-11β-hydroxy-3-oxo-17a-propionyloxy-D-homoandrosta-4,16-dien-17aβ-carbonsäure.

Beispiel 8

In zu Beispiel 7 analoger Weise erhält man aus 11β-hydroxy-3-oxo-17a-propionyloxy-D-homoandrosta-4,16-dien-17aβ-carbonsäure

den 11β-Hydroxy-3-oxo-17a-propionyloxy-D-homoandrosta-4,16-dien-17aβ-carbonsäurechlormethylester, Smp. 192-193°C, $[\alpha]_D$ = −34° (c = 0,1% in Dioxan), UV: $\varepsilon_{241}$ = 15 290.

Beispiel 9

680 mg 11β-Hydroxy-3-oxo-17a-propionyloxy-D-homoandrosta-4,16-dien-17aβ-carbonsäurechlormethylester und 1,36 g Silberfluorid wurden in 8,5 ml Acetonitril 40 Stunden bei 50° gerührt. Nach Zugabe von 50 ml Essigester wurde filtriert, mit Wasser und Kochsalzlösung gewaschen, getrocknet und eingedampft. Aus Äther kristallisierten 297 mg 11β-Hydroxy-3-oxo-17a-propionyloxy-D-homoandrosta-4,16-dien-17aβ-carbonsäurefluormethylester, Smp. 150-151°C, $[\alpha]_D$ −71° (c = 0,1% in Dioxan), UV: $\varepsilon_{240}$ = 15 940.

Beispiel 10

500 mg 17a-Acetoxy-11β-hydroxy-3-oxo-D-homoandrosta-4,16-dien-17aβ-carbonsäuremethylester wurden in 5 ml heissem Äthanol gelöst und nach dem Abkühlen auf 25°C mit 5 ml Orthoameisensäuretriäthylester und 5 mg p-Toluolsulfonsäure versetzt. Nach 15 Minuten wurden 0,05 ml Pyridin zugegeben. Das Reaktionsgemisch wurde mit Methylenchlorid verdünnt, mit Wasser gewaschen, getrocknet und eingedampft. Der erhaltene rohe 17a-Acetoxy-3-äthoxy-11β-hydroxy-D-homoandrosta-3,5,16-trien-17aβ-carbonsäuremethylester wurde bei 0° in 30 ml Aceton mit 240 mg N-Chlorsuccinimid und einer Lösung von 300 mg Natriumacetat und 0,28 ml Essigsäure in 6 ml Wasser versetzt. Nach 30 Minuten wurde das Aceton in Vakuum abgedampft, der Rückstand in Methylenchlorid aufgenommen und mit Natriumhydrogencarbonatlösung und Kochsalzlösung gewaschen. Eindampfen der getrockneten Methylenchloridlösung gab ein Gemisch der Epimeren 17a-Acetoxy-6-chlor-11β-hydroxy-3-oxo-D-homoandrosta-4,16-dien-17aβ-carbonsäuremethylester. Dieses wurde in 25 ml Essigsäure und 5 ml 25% Salzsäure bei 25°C stehen gelassen. Nach 4 Stunden wurde das Lösungsmittel im Vakuum abgedampft. Reinigung des Rückstandes an Kieselgel gab 17a-Acetoxy-6α-chloro-11β-hydroxy-3-oxo-D-homoandrosta-4,16-dien-17aβ-carbonsäuremethylester.

Beispiel 11

17a-Acetoxy-11β-hydroxy-3-oxo-D-homoandrosta-4,16-dien-17aβ-carbonsäuremethylester gibt mit Pyrrolidin in Methanol 17a-Acetoxy-11β-hydroxy-3-(1-pyrrolidinyl)-D-homoandrosta-3,5,16-trien-17aβ-carbonsäuremethylester.

Daraus erhält man bei Behandlung mit Formalinlösung in Methanol und Benzol 17a-Acetoxy-11β-hydroxy-6-hydroxymethyl-3-oxo-D-homoandrosta-4,16-dien-17aβ-carbonsäuremethylester. Mit Salzsäure in Dioxan erhält man daraus 17a-Acetoxy-11β-hydroxy-6-methylen-3-oxo-D-homoandrosta-4,16-dien-17aβ-carbonsäuremethylester. 580 mg 17a-Acetoxy-11β-hydroxy-6-methylen-3-oxo-D-homoandrosta-4,16-dien-17aβ-carbonsäuremethylester und 290 mg Pd/C (5%) wurden in 3 ml Cyclohexen und 30 ml Äthanol am Rückfluss gekocht bis das UV-Spektrum einer entnommenen Probe im Bereich von 290-300 nm keine Absorbtion mehr zeigte. Das Reaktionsgemisch wurde filtriert, mit 15 ml 25%iger Salzsäure versetzt und 1 Stunde stehen gelassen. Nach Abdampfen des Lösungsmittels und Reinigung an Kieselgel erhielt man 17a-Acetoxy-11β-hydroxy-6α-methyl-3-oxo-D-homoandrosta-4,16-dien-17aβ-carbonsäuremethylester.

Beispiel 12

110 mg 17a-Acetoxy-5α-brom-6β-fluor-11β-hydroxy-3-oxo-D-homoandrost-16-en-17aβ-carbonsäuremethylester wurden in 1 ml Eisessig und 0,02 ml 33% HBr in Eisessig 2 Stunden bei 25° gerührt. Das Reaktionsgemisch wurde mit Essigester verdünnt, mit Wasser, Natriumhydrogencarbonatlösung und verdünnter Kochsalzlösung gewaschen, getrocknet und eingedampft. Reinigung des Rohproduktes an Kieselgel gab 17a-Acetoxy-6α-fluoro-11β-hydroxy-3-oxo-D-homoandrosta-4,16-dien-17aβ-carbonsäuremethylester.

Beispiel 13

275 mg 17a-Acetoxy-11β-hydroxy-3-oxo-D-homoandrosta-1,4,16-trien-17aβ-carbonsäuremethylester wurden in 15 ml Aceton mit 0,25 ml Jones-Reagens (26,72 g $CrO_3$, 23 ml konz. $H_2SO_4$, Wasser bis 100 ml Gesamtvolumen) versetzt und bei 25° gerührt. Nach 15 Minuten wurde das Reaktionsgemisch mit 100 ml Wasser versetzt und dreimal mit 30 ml Methylenchlorid extrahiert. Die organischen Phasen wurden einmal mit verdünnter Natriumhydrogencarbonatlösung und einmal mit gesättigter Kochsalzlösung gewaschen und mit Natriumsulfat getrocknet. Nach Entfernung des Lösungsmittels im Vakuum erhielt man 260 mg 17a-Acetoxy-3,11-dioxo-D-homoandrosta-1,4,16-trien-17aβ-carbonsäuremethylester.

Beispiel 14

400 mg 17a-Hydroxy-3,11-dioxo-D-homoandrosta-1,4,16-trien-17aβ-carbonsäuremethylester, 1 ml Essigsäureanhydrid und 1,6 g Dimethylaminopyridin wurden in 20 ml Benzol 36 Stunden am Rückfluss gekocht. Das Reaktionsgemisch wurde mit Äther und Wasser verdünnt. Die Ätherphase wurde mit verdünnter Salzsäure, verdünnter Natriumhydrogencarbonatlösung und gesättigter Kochsalzlösung gewaschen, getrocknet und im Vakuum eingedampft. Chromatographie des Rückstandes an Kieselgel gab 17a-Acetoxy-3,11-dioxo-D-homoandrosta-1,4,16-trien-17aβ-carbonsäuremethylester.

Beispiel 15

600 mg 17a-Acetoxy-3β,11β-dihydroxy-D-homoandrosta-5,16-dien-17aβ-carbonsäuremethylester und 800 mg Aluminium tert.butylat wurden in 8 ml Aceton und 20 ml Benzol am Rückfluss gekocht. Nach 8 Stunden wurde das Reaktionsgemisch mit Essigester verdünnt, mit verdünnter Salzsäure und Wasser gewaschen, getrocknet und im Vakuum eingedampft. Reinigung des Rückstandes an Kieselgel gab 17a-Acetoxy-11β-hydroxy-3-oxo-D-homoandrosta-4,16-dien-17aβ-carbonsäuremethylester, Smp. 229°C.

Beispiel 16

100 mg 17a-Acetoxy-3,11-dioxo-D-homoandrosta-1,4,16-trien-17aβ-carbonsäuremethylester und 30 mg Natriumborhydrid wurden in 3 ml Tetrahydrofuran 5 Stunden bei 30° gerührt. Das Reaktionsgemisch wurde mit Wasser verdünnt und 2 x mit Methylenchlorid extrahiert. Die organischen Lösungen wurden mit Wasser und gesättigter Natriumchloridlösung gewaschen, getrocknet und im Vakuum eingedampft. Präparative Dünnschichtchromatographie gab 17a-Acetoxy-11β-hydroxy-3-oxo-D-homoandrosta-1,4,16-trien-17aβ-carbonsäuremethylester, Smp. 237-238°C.

Beispiel 17

300 mg 17a-Acetoxy-11β-hydroxy-3-oxo-D-ho-

moandrosta-1,4,16-trien-17aβ-carbonsäuremethylester und 300 mg Tris(triphenylphosphin)rhodiumchlorid wurden unter Wasserstoff in 7 ml Benzol und 7 ml Äthanol gelöst und 30 Stunden bei 25°C gerührt. Nach Abdampfen des Lösungsmittels im Vakuum und Chromatographie an Kieselgel erhielt man 17a-Acetoxy-11β-hydroxy-3-oxo-D-homoandrosta-4,16-dien-17aβ-carbonsäuremethylester, Smp. 228-230°C.

Die folgenden Beispiele erläutern die Herstellung von Salben unter Verwendung eines erfindungsgemässen Steroids als aktivem Bestandteil:

Beispiel 18

| | | |
|---|---|---|
| Aktiver Bestandteil | 0,1 | Gew.-% |
| flüssiges Paraffin | 10,0 | Gew.-% |
| weisses weiches Paraffin zur Ergänzung auf | 100 | Gew.-Teile |

Das Steroid wird mit etwas flüssigem Paraffin in der Kugelmühle vermahlen, bis eine Teilchengrösse von weniger als 5 μ erreicht ist. Die Paste wird verdünnt und die Mühle mit dem restlichen flüssigen Paraffinwachs ausgespült. Die Suspension wird zu dem geschmolzenen farblosen weichen Paraffin bei 50°C hinzugegeben und gerührt, bis die Masse kalt ist, wobei man eine homogene Salbe erhält.

Beispiel 19

| | | |
|---|---|---|
| Aktiver Bestandteil | 0,25 | Gew.-% |
| Aluminiumstearat | 3,2 | Gew.-% |
| flüssiges Paraffin zur Ergänzung auf | 100 | Gew.-Teile |

Das Aluminiumstearat wird in flüssigem Paraffin durch Wirbelrühren dispergiert. Die Suspension wird unter weiterem Rühren erwärmt, wobei die Temperatursteigerung mit einer Geschwindigkeit von 2°C pro Minute durchgeführt wird, bis 90°C erreicht sind. Die Temperatur wird bei 90 bis 95°C 30 Minuten lang gehalten, bis sich ein Gel bildet. Dann wird rasch abgekühlt. Der aktive Bestandteil wird auf eine Teilchengrösse von unter 5 μ gemahlen, mit einem geringen Anteil des Gels verrieben und schliesslich in den restlichen Teil des Gels eingearbeitet, wobei eine homogene Mischung erhalten wird.

**Patentansprüche für die Vertragsstaaten:**
BE, CH, DE, FR, GB, IT, LU, NL, SE

1. D-Homosteroide der Formel

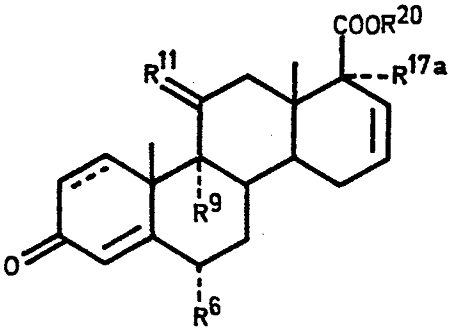

I

in der $R^6$ Wasserstoff, Fluor, Chlor oder Methyl; $R^9$ Wasserstoff, Fluor, Chlor oder Brom; $R^{11}$ Oxo oder (α-H, β-OH) bedeutet, wenn $R^9$ Wasserstoff ist, oder Oxo, (α-H, β-OH), (α-H, β-Fluor) oder (α-H, β-Chlor) bedeutet, wenn $R^9$ = F, Cl oder Br ist, wobei für die Ordnungszahlen der Substituenten $R^9$ und $R^{11}$ von 9,11-Dihalogenverbindungen $R^{11} \leq R^9$ gilt; $R^{17a}$ Acyloxy und $R^{20}$ Niederalkyl oder Halo-niederalkyl und die punktierte 1,2-Bindung eine fakultative C-C-Bindung bedeuten.

2. D-Homosteroide der Formel I gemäss Anspruch 1, dadurch gekennzeichnet, dass $R^9$ Fluor ist.

3. 17aα-Acetoxy-9-fluor-11β-hydroxy-3-oxo-D-homoandrosta-1,4,16-trien-17aβ-carbonsäuremethylester.

4. 11β-Hydroxy-3-oxo-17a-propionyloxy-D-homoandrosta-4,16-dien-17aβ-carbonsäurefluormethylester.

5. D-Homosteroide gemäss einem der Ansprüche 1-4 als pharmazeutische Wirkstoffe.

6. D-Homosteroide gemäss einem der Ansprüche 1-4 als antiinflammatorische Wirkstoffe.

7. Arzneimittel enthaltend ein D-Homosteroid der Formel I nach einem der Ansprüche 1-4.

8. Antiinflammatorisches Mittel enthaltend ein D-Homosteroid der Formel I nach einem der Ansprüche 1-4.

**Ansprüche für den Vertragsstaat:**
AT

1. Verfahren zur Herstellung von neuen D-Homosteroiden der Formel

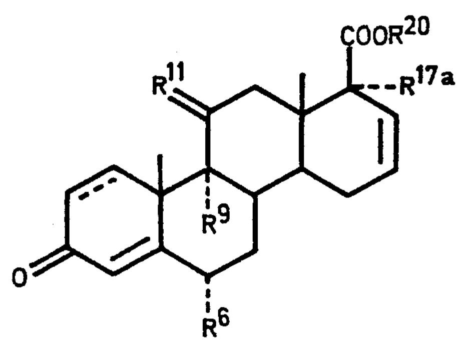

I

in der $R^6$ Wasserstoff, Fluor, Chlor oder Methyl; $R^9$ Wasserstoff, Fluor, Chlor oder Brom; $R^{11}$ Oxo oder (α-H, β-OH) bedeutet, wenn $R^9$ Wasserstoff ist, oder Oxo, (α-H, β-OH), (α-H, β-Fluor) oder (α-H, β-Chlor) bedeutet, wenn $R^9$ = F, Cl oder Br ist, wobei für die Ordnungszahlen der Substituenten $R^9$ und $R^{11}$ von 9,11-Dihalogenverbindungen $R^{11} \leq R^9$ gilt; $R^{17a}$ Acyloxy und $R^{20}$ Niederalkyl oder Halo-niederalkyl und die punktierte 1,2-Bindung eine fakultative C-C-Bindung bedeuten, dadurch gekennzeichnet, dass man die Gruppe $COOR^{201}$ in einem D-Homosteroid der Formel

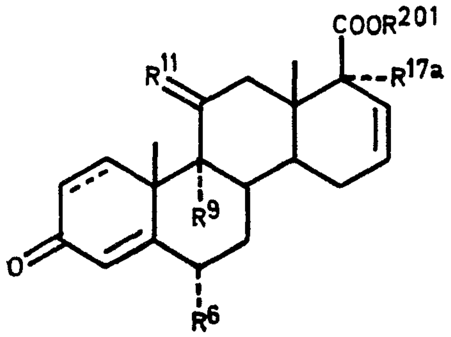

II

worin $R^{201}$ Wasserstoff, Niederalkyl oder Halo-niederalkyl ist und $R^6$, $R^9$, $R^{11}$, $R^{17a}$ und die punktierte 1,2-Bindung die oben angegebenen Bedeutungen haben, funktionell abwandelt.

2. Verfahren zur Herstellung eines $\Delta^{1,4,16}$ oder $\Delta^{4,16}$-D-Homosteroid der Formel I nach Anspruch 1, dadurch gekennzeichnet, dass man ein 1,2-$\Delta^{4,16}$-D-Homosteroid der Formel 1 in 1,2-Stellung dehydriert bzw. ein $\Delta^{1,4,16}$-D-Homosteroid der Formel I hydriert.

3. Verfahren zur Herstellung eines $\Delta^{4,16}$-D-Homosteroids der Formel I, worin $R^6$ Wasserstoff oder Methyl ist, dadurch gekennzeichnet, dass man in einem D-Homosteroid der Formel

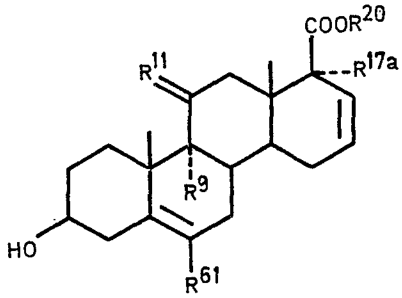

III

worin $R^{61}$ Wasserstoff oder Methyl ist und $R^9$, $R^{11}$, $R^{17a}$ und $R^{20}$ die in Anspruch 1 angegebenen Bedeutungen haben,
die 3-Hydroxy-$\Delta^5$-Gruppierung zur 3-Keto-$\Delta^4$-Gruppierung oxydiert.

4. Verfahren zur Herstellung eines D-Homosteroids der Formel I nach Anspruch 1, worin $R^6$ Methyl, Fluor oder Chlor ist, dadurch gekennzeichnet, dass man ein entsprechendes 6-unsubstituiertes D-Homosteroid in 6-Stellung methyliert, fluoriert oder chloriert und ein erhaltenes 6β-Fluor- oder 6β-Chlor-Isomer gewünschtenfalls zum 6α-Isomer isomerisiert.

5. Verfahren zur Herstellung eines 6-substituierten $\Delta^{4,16}$-D-Homosteroids der Formel I, dadurch gekennzeichnet, dass man ein D-Homosteroid der Formel

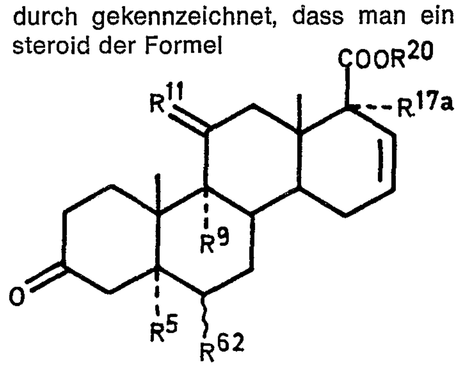

IV

worin $R^5$ Hydroxy, Fluor, Chlor oder Brom und $R^{62}$ Fluor, Chlor oder Methyl ist und $R^9$, $R^{11}$, $R^{17a}$ und $R^{20}$ die im Anspruch 1 angegebenen Bedeutungen haben,
einer $HR^5$-Abspaltung unterwirft.

6. Verfahren zur Herstellung eines 9-substituierten D-Homosteroids der Formel I nach Anspruch 1, worin $R^{11}$ (α-H, β-Hydroxy, β-Fluor oder β-Chlor) ist, dadurch gekennzeichnet, dass man ein D-Homosteroid der Formel

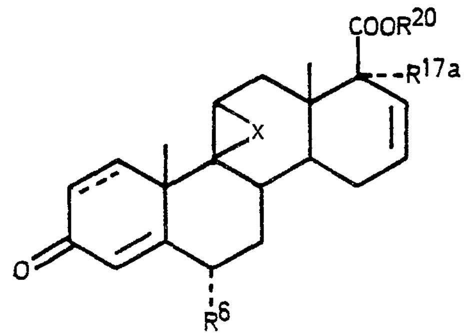

V

worin X eine zusätzliche C-C-Bindung oder ein Sauerstoffatom ist und $R^6$, $R^{17a}$, $R^{20}$ und die punktierte 1,2-Bindung die in Anspruch 1 angegebenen Bedeutungen haben,
mit Chlor, ClF, BrF, BrCl, unterchloriger oder unterbromiger Säure bzw. mit Fluor-, Chlor- oder Bromwasserstoff behandelt.

7. Verfahren zur Herstellung eines 9-unsubstituierten 11-Hydroxy-D-homosteroids der Formel I nach Anspruch 1, dadurch gekennzeichnet, dass man ein entsprechendes 11-Desoxy-D-homosteroid mittels Mikroorganismen oder daraus gewonnenen Enzymen in 11-Stellung hydroxyliert.

8. Verfahren zur Herstellung eines D-Homosteroids der Formel I nach Anspruch 1, worin $R^{11}$ Oxo oder (α-H, β-OH) ist, dadurch gekennzeichnet, dass man ein 11-Hydroxy-D-homosteroid der Formel I oxidiert bzw. ein 11-Oxo-D-homosteroid der Formel I reduziert.

9. Verfahren zur Herstellung eines 17aα-Acyloxy-D-homosteroids der Formel I nach Anspruch 1, dadurch gekennzeichnet, dass man ein entsprechendes 17aα-Hydroxy-D-homosteroid in 17aα-Stellung acyliert.

**Claims for the Contracting states:**
BE, CH, DE, FR, GB, IT, LU, NL, SE

1. D-Homosteroids of the formula

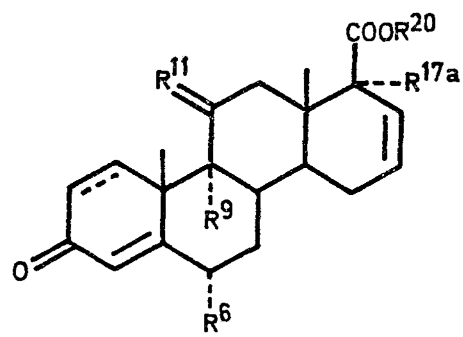

I

in which $R^6$ signifies hydrogen, fluorine, chlorine or methyl; $R^9$ signifies hydrogen, fluorine, chlorine or bromine; $R^{11}$ signifies oxo or (α-H, β-OH) when $R^9$ is hydrogen or signifies oxo, (α-H, β-OH), (α-H, β-fluoro) or (α-H, β-chloro) when $R^9$ is F, Cl or Br, whereby for the atomic numbers of the substituents $R^9$ and $R^{11}$ of 9,11-dihalo compounds $R^{11} \leqslant R^9$ applies; $R^{17a}$ signifies acyloxy and $R^{20}$ signifies lower alkyl or halo-lower alkyl and the dotted 1,2-bond signifies an optional C-C bond.

2. D-Homosteroids of formula I in accordance with claim 1, characterized in that $R^9$ is fluorine.

3. 17aα-Acetoxy-9-fluoro-11β-hydroxy-3-oxo-D-homoandrosta-1,4,16-triene-17aβ-carboxylic acid methyl ester.

4. 11β-Hydroxy-3-oxo-17a-propionyloxy-D-homoandrosta-4,16-diene-17aβ-carboxylic acid fluoromethyl ester.

5. D-Homosteroids in accordance with one of claims 1-4 as pharmaceutically active substances.

6. D-Homosteroids in accordance with one of claims 1-4 as antiinflammatory active substances.

7. Medicament containing a D-homosteroid of formula I according to one of claims 1-4.

8. Antiinflammatory medicament containing a D-homosteroid of formula I according to one of claims 1-4.

**Claims for the contracting state:** AT

1. Process for the manufacture of novel D-homosteroids of the formula

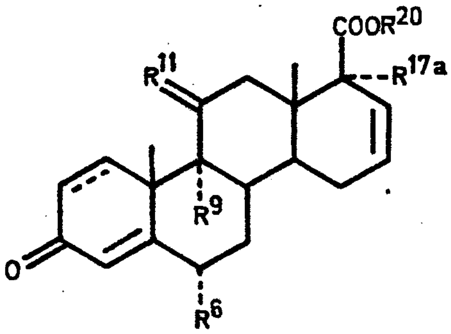

I

in which $R^6$ signifies hydrogen, fluorine, chlorine or methyl; $R^9$ signifies hydrogen, fluorine, chlorine or bromine; $R^{11}$ signifies oxo or (α-H, β-OH) when $R^9$ is hydrogen or signifies oxo, (α-H, β-OH), (α-H, β-fluoro) or (α-H, β-chloro) when $R^9$ is F, Cl or Br, whereby for the atomic numbers of the substituents $R^9$ and $R^{11}$ of 9,11-dihalo compounds $R^{11} \leqslant R^9$ applies; $R^{17a}$ signifies acyloxy and $R^{20}$ signifies lower alkyl or halo-lower alkyl and the dotted 1,2-bond signifies an optional C-C bond,
characterized by functionally modifying the group $COOR^{201}$ in a D-homosteroid of the formula

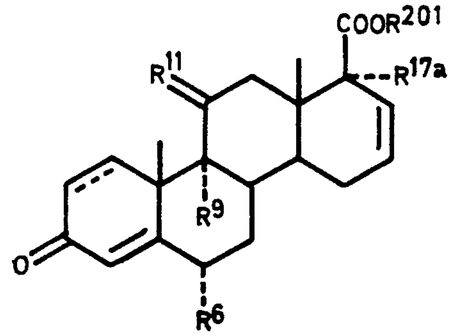

II

wherein $R^{201}$ ist hydrogen, lower alkyl or halo-lower alkyl and $R^6$, $R^9$, $R^{11}$, $R^{17a}$ and the dotted 1,2-bond have the significances given above.

2. Process for the manufacture of a $\Delta^{1,4,16}$ or $\Delta^{4,16}$-D-homosteroid of formula I according to claim 1, characterized by dehydrogenating a 1,2-$\Delta^{4,16}$-D-homosteroid of formula I in the 1,2-position or hydrogenating a $\Delta^{1,4,16}$-D-homosteroid of formula I.

3. Process for the manufacture of a $\Delta^{4,16}$-D-homosteroid of formula I, wherein $R^6$ is hydrogen or methyl, characterized by oxidizing the 3-hydroxy-$\Delta^5$ grouping in a D-homosteroid of the formula

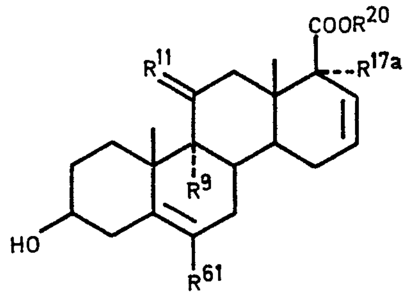

III

wherein $R^{61}$ is hydrogen or methyl and $R^9$, $R^{11}$, $R^{17a}$ and $R^{20}$ have the significances given in claim 1, to the 3-keto-$\Delta^4$ grouping.

4. Process for the manufacture of a D-homosteroid of formula I according to claim 1, wherein $R^6$ is methyl, fluorine or chlorine, characterized by methylating, fluorinating or chlorinating a corresponding 6-unsubstituted D-homosteroid in the 6-position and, if desired, isomerizing a 6β-fluoro- or 6β-chloro-isomer obtained to the 6α-isomer.

5. Process for the manufacture of a 6-substituted $\Delta^{4,16}$-D-homosteroid of formula I, characterized by subjecting a D-homosteroid of the formula

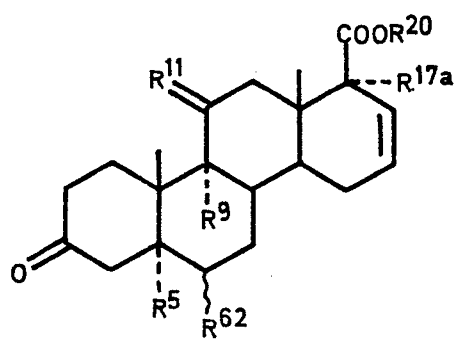

IV

wherein $R^5$ is hydroxy, fluorine, chlorine or bromine and $R^{62}$ is fluorine, chlorine or methyl and $R^9$, $R^{11}$, $R^{17a}$ and $R^{20}$ have the significances given in claim 1, to a $HR^5$-cleavage.

6. Process for the manufacture of a 9-substituted D-homosteroid of formula I according to cleim 1, wherein $R^{11}$ is (α-H, β-hydroxy, β-fluoro or β-chloro), characterized by treating a D-homosteroid of the formula

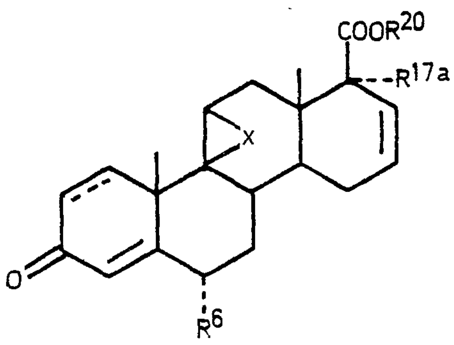

V

wherein X is an additional C-C bond or an oxygen atom and $R^6$, $R^{17a}$, $R^{20}$ and the dotted 1,2-bond have the significances given in claim 1, with chlorine, ClF, BrF, BrCl, hypochlorous or hypobromous acid or with hydrogen fluoride, hydrogen chloride or hydrogen bromide.

7. Process for the manufacture of a 9-unsubstituted 11-hydroxy-D-homosteroid of formula I according to claim 1, characterized by hydroxylating a corresponding 11-desoxy-D-homosteroid in the 11-position by means of microorganisms or enzymes obtained therefrom.

8. Process for the manufacture of a D-homosteroid of formula I according to claim 1, wherein $R^{11}$ is oxo or (α-H, β-OH), characterized by oxidizing a 11-hydroxy-D-homosteroid of formula I or reducing a 11-homosteroid of formula I.

9. Process for the manufacture of a 17aα-acyloxy-D-homosteroid of formula I according to claim 1, caracterized by acylating a corresponding 17aα-hydroxy-D-homosteroid in the 17aα-position.

**Revendications pour les Etats contractants:** BE, CH, DE, FR, GB, IT, LU, NL, SE

1. D-homostéroïdes de formule

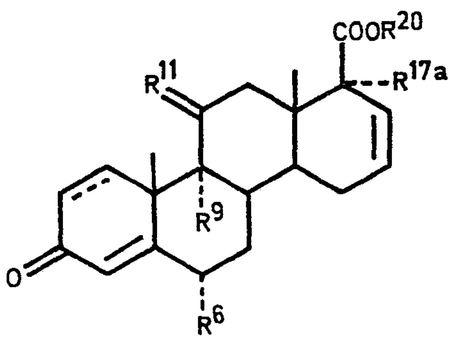

I

où $R^6$ représente un hydrogène, un fluor, un chlore ou un méthyle; $R^9$ représente un hydrogène, un fluor, un chlore ou un brome; $R^{11}$ représente un oxo ou (α-H, β-OH), lorsque $R^9$ est un hydrogène, ou un oxo, (α-H, β-OH), (α-H, β-fluor) ou (α-H, β-chlore), lorsque $R^9$ = F, Cl ou Br où pour le nombre atomique des substituants $R^9$ et $R^{11}$ des composés 9,11-dihalogénés $R^{11} \leqslant R^9$; $R^{17a}$ représente un acyloxy et $R^{20}$ un alcoyle inférieur ou un halo-alcoyle inférieur et la liaison 1,2 pointillée représente une liaison C-C facultative.

2. D-homostéroïdes de formule I selon la revendication 1, caractérisés en ce que $R^9$ ist un fluor.

3. Ester méthylique de l'acide 17a-α-acétoxy-9-fluoro-11-β-hydroxy-3-oxo-D-homo-androsta-1,4,16-trièn-17aβ-carboxylique.

4. Fluorométhylester de l'acide 11β-hydroxy-3-oxo-17a-propionyloxy-D-homoandrosta-4,16-dièn-17aβ-carboxylique.

5. D-homostéroïdes selon l'une des revendications 1-4 comme substances actives pharmaceutiques.

6. D-homostéroïdes selon l'une des revendications 1 à 4 comme substances actives anti-inflammatoires.

7. Médicament contenant un D-homostéroïde de formule I selon l'une des revendications 1-4.

8. Agent anti-inflammatoire contenant un D-homostéroïde de formule I selon l'une des revendications 1-4.

**Revendications pour l'Etat contractant:** AT

1. Procédé de préparation de nouveaux D-homostéroïdes de formule

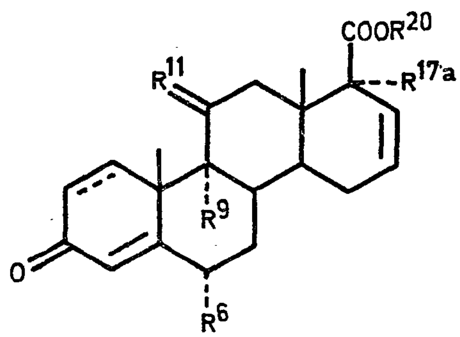

I

où $R^6$ représente un hydrogène, un fluor, un chlore ou un méthyle; $R^9$ représente un hydrogène, un fluor, un chlore ou un brome; $R^{11}$ représente un oxo ou (α-H, β-OH), lorsque $R^9$ est un hydrogène, ou un oxo, (α-H, β-OH), (α-H, β-fluor) ou (α-H, β-chlore), lorsque $R^9$ = F, Cl ou Br où pour le nombre atomique des substituants $R^9$ et $R^{11}$ des composés 9,11-dihalogénés $R^{11} \leqslant R^9$; $R^{17a}$ représente un acyloxy et $R^{20}$ un alcoyle inférieur ou un halo-alcoyle inférieur et la liaison 1,2 pointillée représente une liaison C-C facultative, caractérisé en ce qu'on fait subir une modification fonctionnelle au groupe $COOR^{201}$ dans un D-homostéroïde de formule

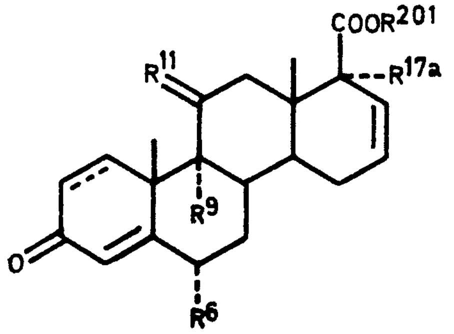

II

où $R^{201}$ représente un hydrogène, un alcoyle inférieur ou un haloalcoyle inférieur et où $R^6$, $R^9$, $R^{11}$, $R^{17a}$ et la liaison 1,2 pointillée ont les significations données ci-dessus.

2. Procédé de préparation d'un $\Delta^{1,4,16}$ ou d'un $\Delta^{4,16}$-D-homostéroïde de formule I selon la revendication 1, caractérisé en ce qu'on déshydrogène en position 1,2 un 1,2-$\Delta^{4,16}$-D-hosmostéroïde de formule I ou en ce qu'on hydrogène un $\Delta^{1,4,16}$-D-homostéroïde de formule I.

3. Procédé de préparation d'un $\Delta^{4,16}$-D-homostéroïde de formule I où $R^6$ est un hydrogène ou un méthyle, caractérisé en ce que, dans un D-homostéroïde de formule

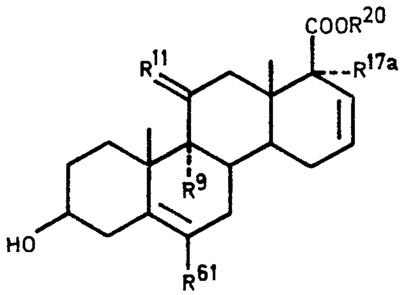

III

où $R^{61}$ est un hydrogène ou un méthyle, et $R^9$, $R^{11}$, $R^{17a}$ et $R^{20}$ ont les significations données dans la revendication 1, on oxyde le groupement 3-hydroxy-$\Delta^5$ en groupement 3-céto-$\Delta^4$.

4. Procédé de préparation d'un D-homostéroïde de formule I selon la revendication 1, où $R^6$ est un méthyle, un fluor ou un chlore, caractérisé en ce qu'on méthyle, en ce qu'on fluore ou en ce qu'on chlore, en position 6 un D-homostéroide correspondant non substitué en 6 et en ce que, si on le désire, on isomérise un isomère 6β-fluoro- ou 6β-chloro obtenu en isomère 6α.

5. Procédé de préparation d'un $\Delta^{4,16}$-D-homostéroïde 6-substitué de formule I, caractérisé en ce qu'on soumet un D-homostéroïde de formule

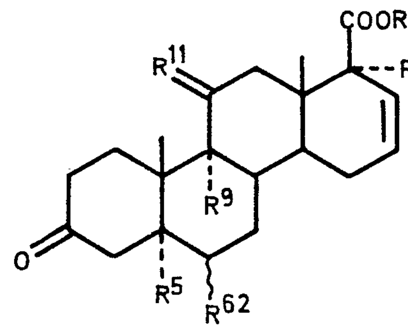

IV

où $R^5$ représente un hydroxy, un fluor, un chlore ou un brome et où $R^{62}$ est un fluor, un chlore ou un méthyle et où $R^9$, $R^{11}$, $R^{17a}$ et $R^{20}$ ont les significations données dans la revendication 1, à une séparation de $HR^5$.

6. Procédé de préparation d'un D-homostéroïde 9-substitué de formule I selon la revendication 1, où $R^{11}$ est (α-H, β-hydroxy, β-fluor ou β-chlore), caractérisé en ce qu'on traite un D-homostéroïde der formule

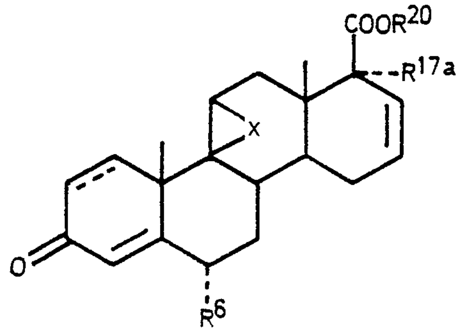

V

où X est une liaison C-C supplémentaire ou un atome d'oxygène et où $R^6$, $R^{17a}$, $R^{20}$ et la liaison 1,2 pointillée ont les significations données dans la revendication 1, avec du chlore, ClF, BrF, BrCl, l'acide hypochloreux ou hypobromeux ou avec l'acide fluorhydrique, l'acide chlorhydrique ou l'acide bromhydrique.

7. Procédé de préparation d'un 11-hydroxy-D-homostéroïde 9-non-substitué de formule I selon la revendication 1, caractérisé en ce qu'on hydroxyle un 11-désoxy-D-homostéroïde correspondant au moyen de microorganismes ou d'enzymes obtenues à partir de ceux-ci, en position 12.

8. Procédé de préparation d'un D-homostéroïde de formule I selon la revendication 1, où $R^{11}$ est un oxo ou (α-H, β-OH), caractérisé en ce qu'on oxyde un 11-hydroxy-D-homostéroïde de formule I ou en ce qu'on réduit un 11-oxo-D-homostéroïde de formule I.

9. Procédé de préparation d'un 17aα-acyloxy-D-homostéroïde de formule I selon la revendication 1, caractérisé en se qu'on acyle un 17α-hydroxy-D-homostéroïde correspondant en position 17aα.